# EUROPEAN PATENT SPECIFICATION

(11) **EP 1 787 625 B1**
(45) Date of publication and mention of the grant of the patent: **26.12.2012**
(21) Application number: 05782232.2
(22) Date of filing: 07.09.2005
(51) Int. Cl.: A23L 1/30, A61K 31/718

(54) **PREVENTIVE/REMEDY FOR OBESITY**
MITTEL ZUR PRÄVENTION/BEHANDLUNG VON ADIPOSITAS
TRAITEMENT PREVENTIF/CURATIF DE L'OBESITE

(30) Priority: 09.09.2004 JP 2004262493
(43) Date of publication of application: 23.05.2007
(73) Proprietor: Kao Corporation, Chuo-ku Tokyo 103-8210 (JP)
(72) Inventor: SHIMOTOYODOME, A., Kao Corp. Research Laboratories, Haga-gun, Tochigi 3213497 (JP); SUZUKI, J., Kao Corp. Research Laboratories, Haga-gun, Tochigi 3213497 (JP); SUGINO, N., Kao Corp. Research Laboratories, Haga-gun, Tochigi 3213497 (JP); NAKAMURA, J., Kao Corp. Research Laboratories, Haga-gun, Tochigi 3213497 (JP)
(74) Representative: HOFFMANN EITLE
(86) International application number: PCT/JP2005/016400
(87) International publication number: WO 2006/028120

(56) References cited:
- EP-A2- 0 648 425
- WO-A1-02/071869
- DE-A1- 3 519 645
- FR-A1- 2 809 928
- JP-A- 4 281 764
- JP-A- 6 049 101
- JP-A- 6 225 719
- JP-A- 7 184 531
- JP-A- 10 279 487
- JP-A- 11 286 497
- JP-A- 59 039 260
- JP-A- 2002 538 846
- JP-A- 2003 310 187
- US-A- 6 099 871
- US-B1- 6 221 420
- SCLAFANI A ET AL: "Starch-induced overeating and overweight in rats: Influence of starch type and form" PHYSIOLOGY AND BEHAVIOR, ELSEVIER SCIENCE LTD., OXFORD, GB, vol. 42, no. 5, 1 January 1988 (1988-01-01), pages 409-415, XP024316873 ISSN: 0031-9384 [retrieved on 1988-01-01]

## Description

### Field of the Invention

The present invention relates to wany corn stearch for use in preventing of treating obesity, and the like.

### Background of the Invention

Recently, incidence of lifestyle-related diseases such as obesity and diabetes is steadily on the rise among Japanese due to lack of exercise in addition to excessive energy intake (an increase in intake of fat and sucrose) resulted from westernized eating habit. Under such social circumstances, taking measures to prevent and remedy obesity and diabetes is of great importance.

For preventing and remedying obesity and diabetes, a typical method suggested by nutritionists is to ingest low-calorie diets or low-fat diets. In recent years, it was reported that each of water-insoluble dietary fibers such as wheat bran, water-soluble dietary fibers such as indigestible dextrin, and resistant starches such as high amylose starch has promoting action of fat excretion (for example, see non-patent document 1), inhibitory action of glucose absorption (for example, see non-patent document 2), reducing action of blood triglyceride (for example, see non-patent document 3), or remedial action of glucose resistance (for example, see non-patent documents 4, 5, and 6). It is believed that they are effective in prevention of and remedy for obesity as well as prevention of and remedy for diabetes.

It is also believed that a sudden increase in blood lipid levels after meals promotes fat accumulation. Hence, for preventing and remedying obesity, an approach to suppress hyperlipidemia (an increase inbloodtriglyceride) after meals is also of great importance. In recent years, as safe and effective fat absorption inhibitors, xanthan gum, propylene glycol alginate (for example, see patent document 1), and chitosan (for example, see patent document 2) were reported.

The above low-calorie diets or low-fat diets can be transiently effective to a small extent in weight loss. However, ingredients composing those diets have simple flavors, so that after a long while people who have been eating them start rejecting them, resulting in difficulty in ingesting such diets for a long period. Also, for ingredients such as the above conventional water-insoluble dietary fibers, water-soluble dietary fibers, and resistant starches to provide the above physiological effects, they should be ingested in high doses and over a long period. Also, even when such physiological effects were obtained, suppression of obesity was not confirmed. In addition, when foods and beverages were made by adding such ingredients, appearance and texture such as taste, crunchiness, and smoothness, which were expected originally from such foods and beverages, were compromised in many cases. Therefore, there were problems in that it was difficult to contain them in sufficient amounts in foods; application areas were limited; and further, it was difficult to keep ingesting such foods and beverages over a long period.

Waxy corn starch, which is derived from waxy corn varieties (waxy corn), is composed of 100% amylopectin. Waxy corn characteristically has great expandability, low gelatinizing temperature, freeze-thaw stability, high transparency, and the like, and is widely used in foods such as rice crackers, packaged rice cakes, rice powder, glutinous rice powder, sauce, and salad dressing. Waxy corn is also widely used in preparation and processing of noodles, frozen foods, snacks, paste foods for improving texture and for providing freezing resistance, transparency of gelatinized solutions, and spinnability (for example, see Non-Patent Document 7).
However, it has not been noted so far that waxy corn starch is effective in preventing and remedying lifestyle-related diseases such as obesity and diabetes.
[Patent Document 1] JP-A-H05-186356
[Patent Document 2] JP-A-H03-290170
[Non-Patent Document 1] Am J Clin Nutr 1978 31 (10 Suppl) : 521-529
[Non-Patent Document 2] Endocrine Journal 1992 68 (6) : 623-35
[Non-Patent Document 3] Am J Clin Nutr 1989 49 (2) : 337-44
[Non-Patent Document 4] Acta Paediatr Hung 1985 26 (1) : 75-7
[Non-Patent Document 5] JEndocrinol 1995 144 (3) : 533-8
[Non-Patent Document 6] Am J Clin Nutr 1989 49 (2) : 337-44
[Non-Patent Document 7] Eiji Fuwa, Toshiaki Komaki, Susumu Hizukuri, Keiji Kainuma, "Denpun Kagaku No Jiten" (in Japanese) (Translation: "Starch Science Encyclopedia"), Asakura Publishing, 2003, p. 503-518
DE 35 19 645 A1 relates to a dietetic foodstuff which is composed of at least 50 % by weight, based on the dry constituents, of a mixture of gluten and carbohydrates digestible by the human body. The gluten delays the uptake of the carbohydrates by the human body. The dietetic foodstuff is suitable for diabetics and the overweight.
Physiology and Behavior, 42, 1988, 409-415 relates to studies on the effect of differences in starch types (hydrolyzed corn starch such as polycose and amylopectin corn starch) and states (gel and powder) on feeding response in rats (amount of food intake and body weight). It is concluded that the relation of starch type to food intake and body weight increase takes the following order polycose gel, amylopectin gel, polycose powder, amylopectin powder.
US 6 099 871 A concerns an infant formula containing certain thickening agents useful for treating regurgitation. The thickening agent can be waxy corn starch, waxy rice starch, or a mixture thereof.
US 6 221 420 B describes foods comprising thermally inhibited starches which are used as food thickeners. Waxy maize is indicated as a possible starch source.
FR 2 809 928 A1 concerns a sponge cake premix comprising a cereal powder of starch and pregelatinized starch. The cereal powder of starch and pregelatinized starch is used instead of flower to improve the texture of the baked product.
EP 0 648 425 A2 concerns a non-fat or low fat edible plastic dispersion which has rheological properties similar to solid margarine or squeezable margarine. The plastic dispersion includes (a) a fat mimetic selected from branched chain amylopectin starch and mixtures thereof with at least one member selected from gelling type maltodextrins, and starch modified by acid hydrolysis to remove amorphous regions therefrom, and (b) inulin and/or gum.
JP 10 279487 A concerns a lipid metabolism improver having activities for reducing neutral fat level in blood plasma and for reducing activity of synthetic enzyme of a fatty acid. The lipid metabolism improver which comprises a starch material containing dietary fiber is obtained by carrying out moist heat treatment of a starch and/or a derivative thereof having 30 wt% or more amylose content, as its active ingredient.
JP 2002 538846 A concerns a ready-to-eat food bar consisting of agglomerated particles and/or flakes of one or more cooked-extruded bases mainly comprising amylaceous materials and milk solids which are coated with a binder mainly comprising sugar, milk solids and a binding agent.

### Disclosure of the invention

The present invention relates to waxy corn starch for use in preventing or treating obesity, the use of waxy corn starch for suppressing fat accumulation in internal organs, the use of waxy corn starch for suppressing blood glucose level increase, the use of waxy corn starch for suppressing blood insulin level increase, the use of waxy corn starch for improving lipid metabolism, and the use of waxy corn starch for promoting fatty acid oxidation.

### Best Mode for Carrying Out the Invention

The present invention relates to providing a raw material for pharmaceuticals and the like, wherein the raw material is effective in preventing and remedying various lifestyle-related diseases such as obesity and hyperlipidemia, safe, and applicable in wide areas.

The present inventors have explored for a raw material that has physical properties different from those of the conventional dietary fibers, such as resistant starches, cellulose, and indigestible dextrin and is also effective in suppressing progression of obesity and diabetes and remedying such diseases, and finally they have discovered that waxy corn starch, exhibiting in a small ingesting amount various physiological actions such as obesity-suppressing action, is useful as a raw material for pharmaceuticals that is effective in preventing and remedying lifestyle-related diseases such as obesity and diabetes.
Waxy corn starch is composed of 100% amylopectin, which is a branched polymer of glucose. Amylopectin is more digestible than amylose, which is a linear polymer of glucose, and, as a result, it is believed that blood glucose levels and blood insulin levels can increase more readily after the ingestion of amylopectin (written by G. H. Anderson, edited by Shuichi Kimura and Akira Adachi, "Glycemic Carbohydrate and Health," Kenpakusha, 2003, p. 45). Therefore, it is surprising indeed that such waxy corn starch has various effects that are useful for preventing and remedying obesity or diabetes, as described above.

The waxy corn starch of the present invention can be effective in prevention of and remedy for various lifestyle-related diseases, for example, in prevention of and remedy for obesity, prevention of and remedy for hyperlipidemia, prevention of cardiac diseases such as cardiac failure, prevention of thrombosis, prevention of colonic cancer or rectal cancer, and hence can be advantageously used mainly as pharmaceuticals and functional foods. In particular, waxy corn starch, is safe for humans, and is also easily gelatinized, so that when it is added to a specially designated food for promoting better health or the like, it will compromise the original texture thereof only at a minimal level.

Waxy corn starch in accordance with the present invention is starch derived from waxy corn varieties (waxy corn). In the present invention, waxy corn starch that is used as a thickener can be used.
As the waxy corn starch in accordance with the present invention, pregelatinized waxy corn starch wherein the above waxy corn starch is pregelatinized, for example, by heat treatment in water can be also used.

Moreover, as described in Examples in the later paragraphs, first, the waxy corn starch has the action of suppressing obesity, by which body weight and fat built up in internal organs are significantly reduced, and hence can be effective, for example, in prevention of hyperlipidemia, prevention of cardiac diseases such as cardiac failure, prevention of thrombosis, and prevention of hypertension, those aliments being caused by obesity. Second, the waxy corn starch has the action of suppressing hyperglycemia or hyperinsulinemia after meals, that is, a sudden increase in blood glucose level and in blood insulin level after meals, as well as the action of suppressing stationary blood glucose levels and blood insulin levels. Third, the waxy corn starch has the action of promoting the activity of oxidizing fatty acids by increasing expression of proteins related to lipid metabolism in the liver, and hence can be effective, for example, in prevention of hyperlipidemia and fatty liver.
Accordingly, the waxy corn starch in accordance with the present invention can be a raw material mainly for pharmaceuticals and functional foods for humans or animals, serving as a preventive/remedy for obesity, an agent for suppressing fat accumulation in internal organs, an agent for suppressing blood glucose level increase, an agent for suppressing blood insulin level increase, an agent for improving lipid metabolism, and an agent for promoting fatty acid oxidation (hereinafter referred to as the preventive/remedy for obesity and the like) .

As for the use of waxy corn starch in preventing or treating obesity and the like in accordance with the present invention, one or more types of the waxy corn starch per se can be solely administered to a human or an animal, and also can be ingested by the addition thereof to various pharmaceuticals, functional foods, pet foods, and the like. Applicable functional foods include foods for promoting beauty, foods for sick people, and specially designated foods for promoting better health, wherein the conceptual targets thereof are physiological functions such as suppression of body fat accumulation and suppression of blood glucose level increase, and such conceptual target will be indicated, if necessary. Applicable pharmaceutical preparations include, for example, oral solid preparations such as tablets and granules, liquids for internal use, and oral liquid preparations such as syrup.
Furthermore, when an oral solid preparation is formed, an excipient, and optionally a binder, a disintegrator, a lubricant, a coloring agent, a flavoring agent, an odor modifier, etc. , are added to the waxy corn starch in accordance with the present invention, and then tablets, coated tablets, granule, powder, capsules, or the like can be formed by the conventional method. Moreover, when an oral liquid preparation is formed, a flavoring agent, a buffer, a stabilizer, a flavoring agent, etc., are added, and then a preparation for internal use, syrup, elixir, or the like can be formed by the conventional method.

The amount of the waxy corn starch to be added in each of the above preparations is generally from 5 to 100% by weight, preferably from 20 to 100% by weight, and more preferably from 30 to 100% by weight.

The dosage (the effective ingesting amount) of the waxy corn stearch for are in preventing as heating obesity and the like in accordance with the present invention is preferably 0.01 g/kg body weight per day or more, more preferably 0.1 g/kg body weight per day or more, and still more preferably 0.4 g/kg body weight per day or more.

### [Examples]

### Test Example 1

The action of suppressing obesity and the action of suppressing blood glucose level increase
A tapioca starch and a waxy corn starch were obtained from National Starch and Chemical Co. The above starch was suspended in distilled water at the final concentration of 50% by weight, which then was autoclaved at 120 °C for 15 min. (steam treatment) followed by freeze-drying to prepare the pregelatinized test starch. A commercial pregelatinized potato starch was obtained from Oriental Yeast Co., Ltd.
Mice were divided into groups, in which each group consistedof 10 mice (C57BL/6J, males, 6weeksold) . The animal diets were prepared by adding each of the pregelatinized starches according to the compositions shown in Table 1. The mice were fed with the above diets. After 24-week feeding, the mice were sacrificed after the blood samples were collected, and the blood glucose level, the blood insulin level, and the fat weight of the internal organs were measured. In Table 2, the body weight, the fat weight of the internal organs, the blood glucose level, and the blood insulin level after 24-week feeding are shown.

**[Table 1]**

| Diet compositions (% by weight) | | |
|---|---|---|
| | Low-fat diet | Test diet (high fat and high sucrose) |
| Pregelatinized test starch | - | 5% |
| Pregelatinized potato starch | 66.5% | 23.5% |
| Sucrose | - | 13% |
| Fat | 5% | 30% |
| Casein | 20% | 20% |
| Cellulose | 4% | 4% |
| Mineral mixture | 3.5% | 3.5% |
| Vitamin mixture | 1% | 1% |

**[Table 2]**

| Mouse body weight, fat weight of the internal organs, blood glucose level, and blood insulin level (non-fasting) after 24-week feeding | | | | |
|---|---|---|---|---|
| | Body weight (g) | Fat weight of the internal organs (g) | Blood glucose level (mg/dL) | Blood insulin level (pg/mL) |
| Low-fat diet | 27.0***** | 1.03***** | 196.5** | 107**** |
| Tapioca starch | 34.4 | 2.67 | 235.3 | 264 |
| Waxy corn starch | 32.1** | 1.91*** | 218.9 | 156** |

| | | | | |
|---|---|---|---|---|
| A statistically significant difference from tapioca starch: **: P < 0.05, ***: P < 0.01, or *****: P < 0.0001 | | | | |

The results shown in Table 2 show that the body weight and the fat weight of the internal organs of the mice that ingested the high-fat diet wherein 5% of tapioca starch was added thereto were higher than those of the mice that ingested the low-fat diet so that the mice that ingested the high-fat diet became obese. However, the body weight and the fat weight of the internal organs of the mice that ingested the diet wherein 5% of waxy corn starch was added thereto were significantly lower than those of the mice that ingested the diet wherein tapioca starch was added thereto. It is thereby shown that waxy corn starch is effective in suppressing obesity.

In addition, the non-fasting blood glucose level and the non-fasting blood insulin level of the mice that ingested the high-fat diet wherein 5% of tapioca starch was added thereto were significantly higher than those of the mice that ingested the low-fat diet. However, the blood glucose level and the blood insulin level of the mice that ingested the diet wherein 5% of waxy corn starch was added thereto were lower than those of the mice that ingested the diet wherein tapioca starch was added thereto. It is thereby shown that waxy corn starch is effective in suppressing the blood glucose level increase as well as effective in remedying hyperinsulinemia.

### Test Example 2

The action of increasing lipid metabolism
Similarly to Test Example 1, each group consisted of 10 mice (C57BL/6J, males, 6 weeks old), and the mice were fed with the animal diets prepared according to the compositions shown in Table 3. After 4-week feeding, the mice were sacrificed and the livers were extirpated. In Table 4, the expression levels of the genes (mRNA) of the proteins related to lipid metabolism in the liver measured by quantitative RT-PCR are shown.

**[Table 3] Diet compositions (% by weight)**

| | Low-fat diet | Test diet (high fat and high sucrose) |
|---|---|---|
| Pregelatinized test starch | - | 10% |
| Pregelatinized potato starch | 66.5% | 18.5% |
| Sucrose | - | 13% |
| Fat | 5% | 30% |
| Casein | 20% | 20% |
| Cellulose | 4% | 4% |
| Mineral mixture | 3.5% | 3.5% |
| Vitamin mixture | 1% | 1% |

**[Table 4]**

| Expression levels of the genes of the proteins related to lipid metabolism in the liver (relative values) | | | |
|---|---|---|---|
| | Expression level of MCAD | Expression level of L-FABP | Expression level of FAT |
| Low-fat diet | 100 | 100 | 100 |
| Tapioca starch | 142 | 81 | 143 |
| Waxy corn starch | 198** | 133** | 227** |

| | | | |
|---|---|---|---|
| A statistically significant difference from tapioca starch: **: P < 0.05 The expression level of each gene was corrected by 36B4 . | | | |

The results shown in Table 4 show that the expression levels of the genes of the proteins related to lipid metabolism in the liver (MCAD, L-FABP, and FAT) in the mice that ingested the diet wherein 10% of waxy corn starch was added thereto were significantly higher than those in the mice that ingested the diet wherein tapioca starch was added thereto.

### Test Example 3

The action of increasing lipid metabolism
Similarly to Test Example 2, mice were divided into groups, in which each group consisted of 20 mice (C57BL/6J, males, 6 weeks old), and the mice were fed with the animal diets prepared according to the compositions shown in Table 3. After 4-week feeding, the mice were sacrificed and the livers were extirpated. In Table 5, the activity of oxidizing fatty acids in the liver measured according to the method previously reported in the publication (Murase. T. et al. Int JObes Relat Metab Disord. 2002 Nov; 26 (11): 1459-64.) is shown.

**[Table 5]**

| Activity of oxidizing fatty acids in the liver | |
|---|---|
| | Activity of oxidizing fatty acids (relative value) |
| Low-fat diet | 100*** |
| Tapioca starch | 131 |
| Waxy corn starch | 168*** |

| | |
|---|---|
| A statistically significant difference from tapioca starch: ***: P < 0.01 | |

The results shown in Table 5 show that the activity of oxidizing fatty acids in the liver in the mice that ingested the diet wherein 10% of waxy corn starch was added thereto were significantly higher than those in the mice that ingested the diet wherein tapioca starch was added thereto. It is thereby shown that lipid metabolism is increased.

## Claims

1. Waxy corn starch for use in preventing or treating obesity.

2. Waxy corn starch for use in suppressing fat accumulation in internal organs.

3. Waxy corn starch for use in suppressing blood glucose level increase.

4. Waxy corn starch for use in suppressing blood insulin level increase.

5. Waxy corn starch for use in improving lipid metabolism.

6. Waxy corn starch for use in promoting fatty acid oxidation.

## Patentansprüche

1. Wachsmaisstärke zur Verwendung zum Verhindern oder Behandeln von Fettleibigkeit.

2. Wachsmaisstärke zur Verwendung im Unterdrücken der Fettanreicherung in inneren Organen.

3. Wachsmaisstärke zur Verwendung im Unterdrücken eines Anstiegs des Blutglukosespiegels.

4. Wachsmaisstärke zur Verwendung im Unterdrücken eines Anstiegs des Blutinsulinspiegels.

5. Wachsmaisstärke zur Verwendung im Verbessern des Lipidmetabolismus.

6. Wachsmaisstärke zur Verwendung im Fördern der Fettsäureoxidation.

## Revendications

1. Amidon de maïs cireux pour son utilisation pour prévenir ou traiter l'obésité.

2. Amidon de maïs cireux pour son utilisation pour supprimer l'accumulation de graisse dans les organes internes.

3. Amidon de maïs cireux pour son utilisation pour supprimer l'augmentation du taux de glycémie.

4. Amidon de maïs cireux pour son utilisation pour supprimer l'augmentation du taux d'insuline dans le sang.

5. Amidon de maïs cireux pour son utilisation pour améliorer le métabolisme lipidique.

6. Amidon de maïs cireux pour son utilisation pour favoriser l'oxydation des acides gras.
